## Europäisches Patentamt
## European Patent Office
## Office européen des brevets

(19)

(11) Publication number: **0 039 458**
**A1**

(12)
# EUROPEAN PATENT APPLICATION

(21) Application number: 81103117.8

(51) Int. Cl.³: **G 03 B 17/48, A 61 B 1/04**

(22) Date of filing: 25.04.81

(30) Priority: 02.05.80 JP 58987/80

(71) Applicant: **OLYMPUS OPTICAL CO., LTD.,**
**43-2, 2-chome, Hatagaya Shibuya-ku, Tokyo (JP)**

(43) Date of publication of application: 11.11.81
**Bulletin 81/45**

(72) Inventor: **Hosada, Seiichi, 4-32-8, Honmachi, Fuchu-shi**
**Tokyo (JP)**

(84) Designated Contracting States: **AT BE CH DE FR GB IT**
**LI NL SE**

(74) Representative: **Kempe, Wolfgang, Dr., Postfach 1273,**
**D-6800 Mannheim 1 (DE)**

(54) Light supply device for endoscope.

(57) Light flash from a photoflash tube (36) is adapted to be coupled through a light guide fiber cable (16) of an endoscope to illuminate an object (50), and light reflected from the object (50) is adapted to be projected onto a film (24) for exposure. A photo-electric signal corresponding to the amount of exposure of the film (24) is integrated by an integrator (46), which is set simultaneously with the start of flash-radiation of the photoflash tube (36), that is, the integrating operation of the integrator (46) is started simultaneously with the start of flash-irradiation of the photoflash tube (36).

- 1 -

Light supply device for endoscope

This invention relates to light supply devices for endoscope and, more particularly, to endoscope light supply devices having an automatic exposure control circuit.

As the light supply device for endoscope, there is one, in which an observation light source and a photographing light source are provided such that their light paths with respect to an endoscope image guide are switched from one to the other. In such a light supply device for endoscope, the photographing light source is flashed after the switching of the light paths is effected in response to the closure of a synchronous contact switch provided in the camera. In other words, there is a delay time involved from the closure of the synchronous contact switch till the flashing of the light source. On the other hand, the integrating operation of an integrator provided in an automatic exposure control circuit is started with the closure of the synchronous contact switch. Therefore, during the delay time mentioned above undesired integration is liable to be effected due to a drift in the integrator. In this case, accurate integration output can no longer be obtained, making it impossible to obtain automatic exposure control of the proper exposure. Particularly, in the case of the light source device of the type switching the light paths, where an FP switch, i.e., a switch which is closed before the opening of the shutter, is used as the synchronous contact switch, the aforementioned delay time is long that much, so that the integration is greatly affected by the drift. It has been thought to use a low-drift operational amplifier for suppressing the adverse effects of the drift, but such an operational amplifier is expensive.

An object of the invention, accordingly, is to provide a light supply device for endoscope, which permits accurate automatic right exposure control and can be provided at a low cost.

In the light supply device for endoscope according to the invention a delay circuit for providing an output signal a predetermined period of time after the operation of a synchronous contact switch is provided, and a photoflash tube is caused to provide flashlight in synchronism to the output signal of the delay circuit while at the same time starting the calculation of an exposure calculating circuit for calculating the exposure mount. The delay time of the delay circuit is set to a period from the start of operation of the synchronous contact switch till the instant when the shutter is fully opened.

This invention can be more fully understood from the following detailed description when taken in conjunction with the accompanying drawings, in which:

Fig. 1 shows a schematic diagram of an endoscope system using a light supply device according to an embodiment of the invention; and

Fig. 2 is a time chart for explaining the operation of the light supply device of Fig. 1.

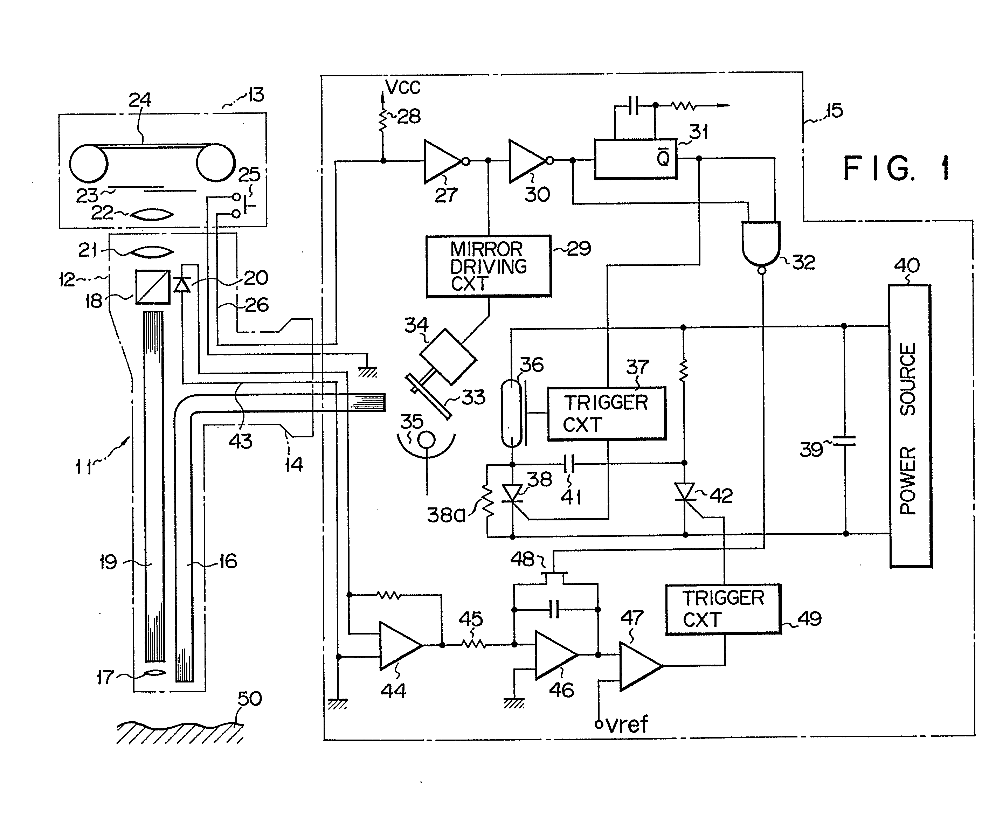

Fig. 1 shows an endoscope system. In this system, an endoscope 11 has an eyepiece section 12, on which an endoscope camera 13 is detachably mounted, and also has a connector 14 which is connected to a light supply device 15. In the endoscope 11, a light guide fiber cable 16 extending into the light guide device 15 and an image guide fiber cable 19 extending from an objective lens 17 to a beam splitter 18 are provided. A light receiving element (i.e., a photodiode) 20 is provided on one side of the beam splitter 18. An endoscope eyepiece lens 21 is provided to face the corresponding end of the image guide fiber cable 19 via a beam splitter 18. The endoscope camera 13 includes a camera objective lens 22, a shutter 23 and a film 24 provided in the mentioned order with respect to the endoscope eyepiece lens 21. It further includes a synchronous contact switch 25, which is connected to the input terminal of an inverter 27 in the light supply device 15 by a signal line 26 extending through the endoscope 11. To the input terminal of the inverter 27, a voltage $V_{cc}$ is applied through a resistor 28. The output terminal of the inverter 27 is connected to a mirror driving circuit 29 and also connected through an inverter 30 to the input terminal of a monostable multivibrator 31. The output terminal of the inverter 30 and also the output terminal of the multivibrator 31 are connected to respective input terminals of a NAND gate 32. The mirror driving circuit 29 is connected to a mirror driving solenoid 34, which drives a mirror 33 disposed at an angle of, for instance, 45 degrees with respect to the light axis of the light guide fiber cable 16. An observation lamp (i.e., a halogen lamp) 35 is provided to face the mirror 33, and a photoflash tube 36 is provided on the light axis of the light guide fiber cable 16. The photoflash tube 36 has a trigger electrode connected to the output terminal of the trigger circuit 37, and also it is connected through a main thyristor 38 parallel-connected with a resistor 38a to a power source 40, and a main capacitor 39 is connected thereacross. The main thyristor 38 has its gate connected to the output terminal of the trigger circuit 37, and the output terminal of the multivibrator 31 is connected to the input terminal of the trigger circuit 37. A quenching circuit constituted by a capacitor 41 and a thyristor 42 is connected between the anode and cathode of the main thyristor 38.

The light receiving element 20 in the endoscope 11 is connected to the input terminal of an amplifier 44 of the light supply device 15 by a signal line 43. The output terminal of the amplifier 44 is connected through a resistor 45 to an integrator 46 for calculating the exposure. The output terminal of the integrator 46 is connected to a comparator 47. The output terminal of the NAND gate 32 mentioned above is connected to an analog switch 48 of the integrator 46, and the output terminal of the comparator 46 is connected to the input terminal of a trigger circuit 49, the output of which is connected to the gate of the thyristor 42.

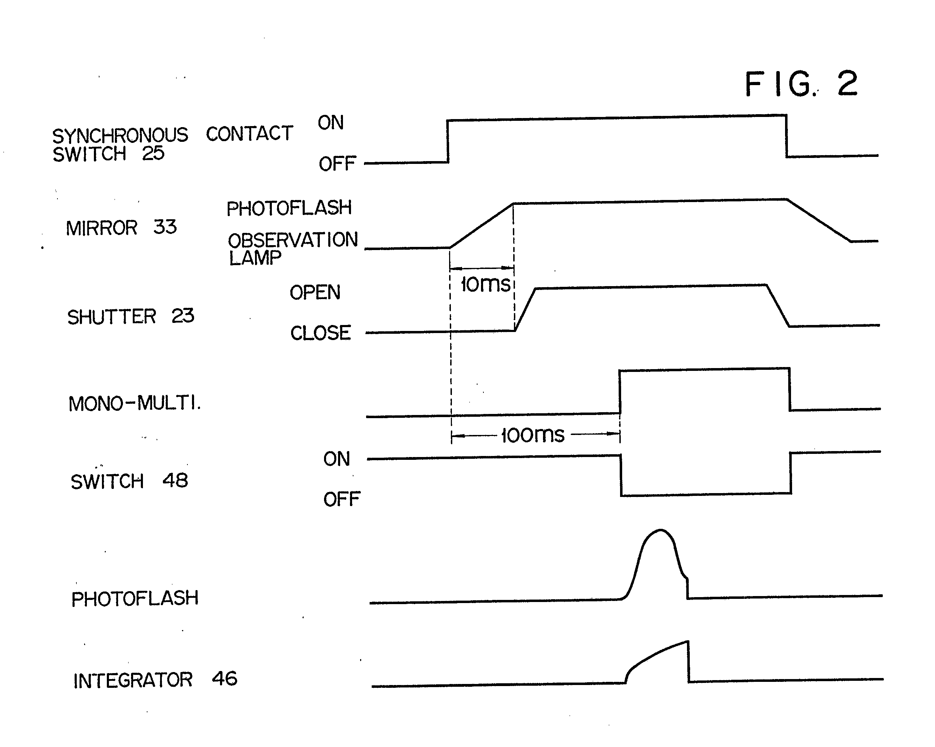

The operation of the endoscope system described above will now be described with reference to the time chart of Fig. 2. When the power source 40 of the light supply device 15 is closed, the main capacitor 39 and capacitor 41 of the quenching circuit are charged. Also at this time, the observation lamp 35 is lit, and light therefrom is led by the mirror 33 into the light guide fiber cable 16 for illuminating an object 50. In this state, the object 50 can be observed through an eyepiece lens (not shown) of the camera 13. When a shutter release button (not shown) is depressed in this state for taking an endoscopic image of the object 50, the synchronous contact switch 25 is turned on, and a shutter release signal is provided from the inverter 27. In response to this shutter release signal, the mirror driving circuit 29 energizes the mirror driving solenoid 34, whereby the mirror 33 begins to switch the light path of the observation lamp 35 to the photoflash path.

- 5 -                    0039458

The observation lamp 35 may be extinguished at this time.  The shutter release signal is coupled through the inverter 30 to the monostable multivibrator 31.  The monostable multivibrator 31 is constructed to provide a delay time of about 100 msec., so that its output is not changed at this time.  The switching of the light paths is completed in about 10 msec. from the instant when the synchronous contact switch 25 is turned on.  As soon as the switching is ended, the shutter 23 begins to be opened.  After the shutter 23 is completely opened, the output of the monostable multivibrator 31 is changed to a high level, whereupon the trigger circuit 37 is driven by this output signal to supply a trigger signal to the trigger electrode 13 of the photoflash tube 36 and the gate electrode of the main thyristor 38.  As a result, the photoflash tube 36 radiates a light flash.  At the same time, the output of the NAND gate 32 is changed to a low level to turn off the analog switch 48, thus causing the integrator 46 to commence integration. The light flash from the photoflash tube 36 is coupled through the light guide fiber cable 16 to illuminate the object 50, and light reflected thereby is coupled through the objective lens 17 and image guide fiber cable 19 to be incident on the beam splitter 18.  A major portion of the light incident on the beam splitter 18 is coupled through the endoscope eyepiece lens 21, objective lens 22 of the camera 13 and shutter 23 to the film 24 to effect the exposure thereof.  On the other hand, light coupled through the beam splitter 18 to the light receiving element 20 is converted into a photoelectric signal, which is supplied through the signal line 43 to the amplifier 44 in the light supply device 15.  After being amplified by the amplifier 44, the photoelectric signal is coupled through the resistor 45 to the integrator 46 for integration.  It is to be understand that the integrator 46 starts the operation of integrating the amplified photoelectric signal simultaneously with the start of flash radiation of the photoflash tube 36. The output voltage of the integrator 46, resulting from the integration, is compared with a reference voltage $V_{ref}$, and when the integration output voltage reaches the reference voltage $V_{ref}$, the comparator 47 provides an output to drive the trigger circuit 49, thus causing a trigger signal to be supplied therefrom to the thyristor 42 of the quenching circuit. As a result, the thyristor 42 is turned on to cause the main thyristor 38 to be reversely biased by the voltage across the capacitor 41 of the quenching circuit, whereby the flash-radiation of the photoflash tube 26 is interrupted. After a predetermined subsequent period of time, the shutter of the camera 13 is closed to open the synchronous contact switch 25. As a result, the output of the AND gate 32 is changed to the high level to turn on the analog switch so as to reset the integrator 46. Also, the mirror 33 is restored to the initial position to permit light from the observation light source 35 to be coupled to the light guide fiber cable 16 again, that is, the observation state of the endoscope system is recovered.

As has been described in the foregoing, according to the invention the integrating operation of the integrator does not take place for a period of about 100 msec. from the closure of the synchronous contact switch till the start of flash-radiation of the photoflash tube after the complete opening of the shutter, and there is no possibility of the charging of the integration capacitor due to the drift during this period. Thus, it is possible to eliminate the exposure error due to drift. While drift occurs during the flash-radiation of the photoflash tube, it can be ignored for the automatic exposure control effected for the period of flash-radiation of the photoflash tube which is as short as 10 msec.  Further, since there is
no need of using a low-drift operation amplifier to
the end of preventing the effects of the drift, the
construction can be inexpensively provided.

Claims:

1.   A light supply device for endoscope including an observation light source (35) and a photographing light source (36) characterized by comprising means (29, 33, 34) for changing an operation from an observation to a photographing, a delay circuit (31) for providing an output signal after a predetermined time necessary for changing from the observation to the photographing, in response to the operation of a synchronous contact switch (25) of a camera (13), means (37) for causing said photographing light source to irradiate a light flash in response to the output signal of the delay circuit, an exposure amount calculating means (46, 47) for calculating the amount of exposure of a camera film and providing an exposure stop signal when a predetermined exposure amount is detected, means (32, 48) for starting the calculating operation of said exposure amount calculating means (46, 47) simultaneously with the start of the light flash of said photographing light source, and means for interrupting the light flash of said photographing light source (36) in accordance with the exposure stop signal from said exposure amount calculating means.

2.   The light supply device according to claim 1, characterized in that said exposure amount calculating means includes an integrator (46) for integrating a photoelectric signal corresponding to the amount of exposure of the film and a comparator (47) for comparing the integration output of said integrator and a reference value, and said calculation starting means includes a switching element (48) for setting said integrator (46) in response to the output signal of said delay circuit (31).

3.   The light supply device according to claim 1 or 2, characterized in that said delay circuit is a monostable multivibrator (31) for providing an output signal a predetermined period of time after the operation of said synchronous contact switch (25).

4. The light supply device according to claim 3, characterized in that said monostable multivibrator (31) provides a delay time of 100 msec.

5. The light supply device according to claim 2, characterized in that said photographing light source is a photoflash device (36).

6. The light supply device according to claim 1 or 2, characterized in that said observation light source is a halogen lamp (35).

FIG. 1

FIG. 2

SYNCHRONOUS CONTACT SWITCH 25 — ON / OFF

MIRROR 33 — PHOTOFLASH / OBSERVATION LAMP — 10ms

SHUTTER 23 — OPEN / CLOSE

MONO-MULTI. — 100ms

SWITCH 48 — ON / OFF

PHOTOFLASH

INTEGRATOR 46

2/2

0039458

## DOCUMENTS CONSIDERED TO·BE RELEVANT

| Category | Citation of document with indication, where appropriate, of relevant passages | Relevant to claim. |
|---|---|---|
| | DE – B – 2 041 054 (OLYMPUS OPTICAL) <br> * complete document * <br> -- | 1,2 |
| | DE – B2 – 2 065 710 (OLYMPUS OPTICAL) <br> * complete document * <br> -- | 1,2 |
| E | EP – A1 – 0 027 605 (OLYMPUS OPTICAL) <br> * claims 1 to 6; fig. 1 * <br> -- | 1,2 |
| P | EP – A1 – 0 018 126 (OLYMPUS OPTICAL) <br> * fig. 1 * <br> -- | 1 |
| A | DE – A – 1 951 226 (OLYMPUS OPTICAL) <br> * fig. 1 * <br> ---- | |

**CLASSIFICATION OF THE APPLICATION (Int. Cl.³)**

G 03 B 17/48
A 61 B 1/04

**TECHNICAL FIELDS SEARCHED (Int. Cl.³)**

G 03 B 17/00
A 61 B 1/00

**CATEGORY OF CITED DOCUMENTS**

X: particularly relevant
A: technological background
O: non-written disclosure .
P: intermediate document
T: theory or principle underlying the invention
E: conflicting application
D: document cited in the application
L: citation for other reasons

&: member of the same patent family, corresponding document

X | The present search report has been drawn up for all claims

| Place of search | Date of completion of the search | Examiner |
|---|---|---|
| Berlin | 04-08-1981 | HOPPE |

EPO Form 1503.1   06.78